# EUROPÄISCHE PATENTSCHRIFT

(11) **EP 0 763 061 B1**
(45) Veröffentlichungstag und Bekanntmachung des Hinweises auf die Patenterteilung: **30.10.2002**
(21) Anmeldenummer: 95921780.3
(22) Anmeldetag: 30.05.1995
(51) Int. Cl.: C07K 14/58, A61K 38/22

(54) **VERFAHREN ZUR HERSTELLUNG VON CARDIODILATIN-FRAGMENTEN, HOCHGEREINIGTE CARDIODILATIN-FRAGMENTE UND ZWISCHENPRODUKTE ZU DEREN HERSTELLUNG**
PROCESS AND INTERMEDIATE PRODUCTS FOR PREPARING CARDIODILATIN FRAGMENTS, AND HIGHLY PURIFIED CARDIODILATIN FRAGMENTS
PROCEDE ET PRODUITS INTERMEDIAIRES UTILES POUR PREPARER DES FRAGMENTS DE CARDIODILATINE, ET FRAGMENTS TRES PURS DE CARDIODILATINE

(30) Priorität: 02.06.1994 DE 4420381; 10.04.1995 DE 19513784
(43) Veröffentlichungstag der Anmeldung: 19.03.1997
(73) Patentinhaber: Forssmann, Wolf-Georg, Prof. Dr. med., 30625 Hannover (DE)
(72) Erfinder: IMMER, Hansueli, CH-4710 Balsthal (CH); FORSSMANN, Wolf-Georg, D-30175 Hannover (DE); ADERMANN, Knut, D-30177 Hannover (DE); KLESSEN, Christian, D-67742 Lauterecken (DE)
(74) Vertreter: Meyers, Hans-Wilhelm, Dr.Dipl.-Chem.
(86) Internationale Anmeldenummer: EP9502050
(87) Internationale Veröffentlichungsnummer: WO95033769

(56) Entgegenhaltungen:
- EP-A- 0 140 731
- EP-A- 0 173 557
- EP-A- 0 369 474
- WO-A-85/04870
- WO-A-88/06596
- N.L.JOHANSEN ET AL. 'Peptides, Proc.Eur.Pept.Symp. 19th' 1987 , DE GRUYTER , BERLIN, BRD 19 siehe Seite 239-242 siehe Seite 241, letzter Absatz

### SEQUENZPROTOKOLL

(1) ALLGEMEINE ANGABEN:
   (i) ANMELDER:
      (A) NAME: Forssmann, Wolf-Georg, Prof. Dr. med. Dr. h.c.
      (B) STRASSE: Bluecherstr. S
      (C) ORT: Hannover
      (E) LAND: Deutschland
      (F) POSTLEITZAHL: 30175

      (A) NAME: Adermann, Knut, Dr.
      (B) STRASSE: Schleidenstr. 5
      (C) ORT: Hannover
      (E) LAND: Deutschland
      (F) POSTLEITZAHL: 30177

      (A) NAME: Immer. Hans-Ueli, Dr.
      (B) STRASSE: Hasenweg 6
      (C) ORT: Balsthal
      (E) LAND: Schweiz
      (F) POSTLEITZAHL: 4710

      (A) NAME: Klessen, Christian, Dr.
      (B) STRASSE: Hauptstr. 26
      (C) ORT: Lauterecken
      (E) LAND: Deutschland
      (F) POSTLEITZAHL: 67742
   (ii) BEZEICHNUNG DER ERFINDUNG: Verfahren zur Herstellung von Cardiodilatin-Fragmenten, hochgereinigte Cardiodilatin-Fragmente und Zwischenprodukte zu deren Herstellung
   (iii) ANZAHL DER SEQUENZEN: 3
   (iv) COMPUTER-LESBARE FASSUNG:
      (A) DATENTRÄGER: Floppy disk
      (B) COMPUTER: IBM PC compatib1e
      (C) BETRIEBSSYSTEM: PC-DOS/MS-DOS
      (D) SOFTWARE: Patentln Release #1.0, Version »1.30 (EPA)
   (vi) DATEN DER URANMELDUNG:
      (A) ANMELDENUMMER: DE P 44 20 381.0
      (B) ANMELDETAG: 02-JUN-1994
   (vi) DATEN DER URANMELDUNG:
      (A) ANMELDEMUMMER: DE 195 13 784.1
      (B) ANMELDETAG: 10-APR-1995
(2) ANGABEN ZU SEQ ID NO: 1:
   (i) SEQUENZKENNZEICHEN:
      (A) LANGE: 17 Aminosäuren
      (B) ART: Aminosäure
      (C) STRANGFORM:
      (D) TOPOLOGIE: linear
   (ii) ART DES MOLEKÜLS: Peptid
   (xi) SEQUENZBESCHREIBUMG: SEQ ID NO: 1:
   Cys Phe Gly Gly Arg Met Asp Arg Ile Gly Ala Gln Ser Gly Leu Gly
   1               5                   10                  15
   Cys
(2) ANGABEN ZU SEQ ID NO: 2:
   (i) SEQUENZKENNZEICHEN:
      (A) LANGE: 15 Aminosäuren
      (B) ART: Aminosäure
      (C) STRANGFORM:
      (D) TOPOLOGIE: linear
   (ii) ART DES MOLEKÜLS: Peptid
   (Xi) SEQUENZBESCHREIBUNG: SEQ ID NO: 2:
   Leu Arg Ala Leu Leu Thr Ala Pro Arg Ser Leu Arg Arg Ser Ser
   1               5                   10                  15
(2) ANGABEN ZU SEQ ID NO: 3:
   (i) SEQUENZKENNZEICHEN:
      (A) LANGE: 5 Aminosäuren
      (B) ART: Aminosäure
      (C) STRANGFORM:
      (D) TOPOLOGIE: linear
   (ii) ART DES MOLEKÜLS: Peptid
   (xi) SEQUENZBESCHREIBUNG: SEQ ID NO: 3:
   Asn Ser Phe Arg Tyr
   1               5

## Beschreibung

Die Erfindung betrifft ein Verfahren zur Herstellung von Cardiodilatin Fragmenten, hochgereinigte Cardiodilatin-Fragmente und entsprechende Zwischenprodukte zur Herstellug dieser Fragmente.

Gegenstand der vorliegenden Erfindung ist ein Verfahren zur Herstellung von Cardiodilatin gemäß Patentanspruch 1.

Cardiodilatin ist ein Peptid aus der Familie der natriuretischen Peptide. Diese Peptide spielen eine wichtige Rolle in der Regulierung des Salz- und Wasserhaushaltes im Körper. Der Prototyp der natriuretischen Hormone ist das Cardiodilatin, das in der Literatur auch als atriales natriuretisches Peptid (CDD/ANP) bezeichnet wird. Die Isolierung von Cardiodilatin sowie die Herstellung von biologisch aktiven Fragmenten des Cardiodilatins sind aus der US-Patentschrift 4,751,284 bekannt (s.a. W.G. Forssmann et al., Kim Wochenschr, 1986, 64 (Suppl. VI), 4-12). Eine Übersicht über die Isolierung und Charakterisierung von Cardiodilatin und dessen Fragmente, sowie deren physiologischen Eigenschaften ist in Eur J Clin Invest 1986, 16: 439-451 (W.G. Forssmann) veröffentlicht. Ein spezielles Fragment des Cardiodilatins mit einer Kettenlänge von 32 Aminosäuren ist aus der EP 0,349,545 bekannt. Dieses Fragment wird in der Literatur mittlerweile auch als Urodilatin (INN: Ularitide) bezeichnet. Weiterhin beschreibt US 5,354,900 (Suntory) ein biologisch aktives Fragment mit einer Kettenlänge von 28 Aminosäuren, das als α-hANP bekannt ist. Weitere biologisch aktive Cardiodilatin-Fragmente oder Derivate davon sind in EP 0,180,615 beschrieben. Insbesondere werden dort Cardiodilatin-Fragmente beschrieben, die am N-Terminus mit der Aminosäureposition Arg¹⁰² beginnen und mit der Aminosäureposition Arg¹²⁵ oder Arg¹²⁶ am C-Terminus enden. In der Literatur wird anstelle der Bezeichnung Cardiodilatin häufig auch die Bezeichnung Atriales Natriuretisches Peptid (ANP) verwendet. Bei der im folgenden verwendeten Numerierung der Sequenzen der Aminosäuren des Cardiodilatins wird auf die in EP 0,349,545 verwendete Nomenklatur für das Peptid ANF/CDD (1-126) (=ANP) bezug genommen.

Gemeinsames Strukturmerkmal von allen bisher bekannten biologisch aktiven Cardiodilatin-Fragmenten ist die Ausbildung einer Disulfid-Brücke zwischen den Aminosäuren Cys¹⁰⁵ und Cys¹²¹, wobei ein stabiler Ring von 17 Aminosäuren entsteht. Es wird angenommen, daß die Ausbildung dieses Ringes für die biologische Wirkung der Cardiodilatin-Derivate maßgeblich verantwortlich ist. Die Cardiodilatin-Fragmente sind an der Position Cys¹⁰⁵ durch eine Aminosäurekette R¹ mit einer Kettenlänge von 0 - 15 Aminosäuren und an der Position Cys¹²¹ durch eine Kette R² mit einer Kettenlänge von 0 - 5 Aminosäuren substituiert. Der Kernbereich ANP(105-121) ist in der [SEQ ID NO. 1] in linearisierter Form angegeben.

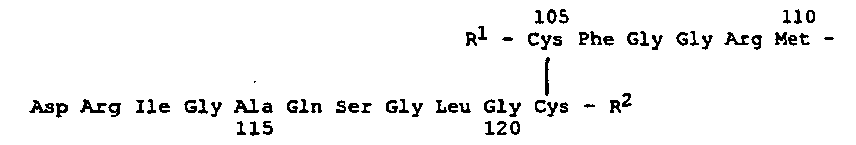

Das Cardiodilatin-Fragment ANP(95-126) mit der INN-Bezeichnung Ularitide ist ein besonders stabiles und biologisch aktives Humanpeptid mit diuretischer Wirkung und relaxierender Wirkung auf die glatte Gefäßmuskulatur, das aus 32 Aminosäuren aufgebaut ist und folgende Sequenz besitzt, wobei die beiden Cystein-Aminosäuren in Position 11 und 27 des Peptids eine Disulfidbrücke ausbilden:

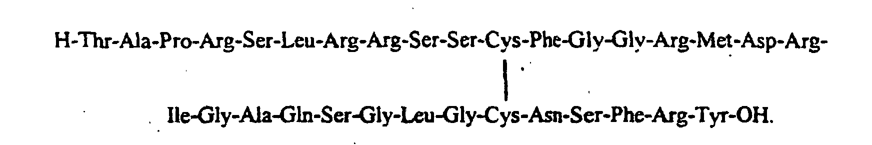

Urodilatin kommt im menschlichen Urin vor. EP 0 349 545 beschreibt ein Verfahren zur Gewinnung von Urodilatin aus dem Urin mittels Alginsäure, in dem die an der Alginsäure adsorbierten Peptide eluiert werden, das Eluat nach üblichen Reinigungsmethoden fraktioniert und die aktive Fraktion mittels eines Tests, der auf der Untersuchung der relaxierenden Wirkung des Urodilatins auf die glatte Muskulatur basiert, gewonnen wird.

EP 0 349 545 beschreibt weiterhin die stufenweise chemische Synthese von Urodilatin nach dem Merrifield-Verfahren (J Am Chem Soc 1963, 85: 2149-2156) an einer Festphase gemäß dem ABI-Standard-Programm nach der Boc-Strategie. Daneben wird in dieser Patentschrift die Herstellung von Urodilatin aus dem Teilfragment ANP(99-126) beschrieben. Dieses Fragment liegt an einer Festphase gebunden vor und wird mit einem zweiten Teilfragment, dem Tetrapeptid Boc-Thr(But)-Ala-Pro-Arg(Tos), umgesetzt. Das aus der Kondensation erhaltene Peptid ANP(95-126) wird vom Träger abgespalten und nach Abspaltung der Schutzgruppen cyclisiert, und anschließend in an sich bekannter Weise aufbereitet und gereinigt.

In EP 0,180,615 wird ebenfalls die chemische Synthese mittels eines festen Trägers beschrieben, wobei der Aufbau der dort beschriebenen Cardiodilatin-Fragmente suksessive vom C-Terminus aus in einer Richtung zum N-Terminus erfolgt. Eine Kondensation über Teilfragmente wird hierbei nicht beschrieben.

Die nach den in der Literatur beschriebenen Verfahren hergestellten Cardiodilatin-Fragmente besaßen jedoch nicht die für klinische Studien und für die Zulassung als Arzneimittel notwendige Reinheit, da aufgrund der Synthese Peptidverunreinigungen in das Endprodukt eingeschleust wurden, die auch durch nachträgliche Reinigungsverfahren nicht mehr entfernt werden konnten. Die Verunreinigungen können aufgrund ihrer immunogenen Eigenschaften bei der Verabreichung am Patienten zu unerwünschten Nebenwirkungen führen, so daß die therapeutische Anwendung mit Risiken behaftet war. Außerdem war die Synthese nur in kleinem Maßstab mit vertretbarem technischem Aufwand realisierbar und für eine Produktion in größeren Mengen wirtschaftlich ungeeignet. Ein weiterer Nachteil der bekannten Syntheseverfahren war ferner das vorhandene potentielle Racemisierungsrisiko, wodurch das Urodilatin in geringerer Reinheit, geringerer biologischer Aktivität und ungenügender Ausbeute erhalten wurde. Die bei den bisherigen Synthesen oft erfolgende Racemisierung des Produktes führte häufig zu einer ungenügenden optischen Reinheit des Endproduktes, wobei diese Verunreinigungen häufig nicht bzw. nur mit sehr hohem technischen Aufwand wieder entfernt werden können.

EP-A-173 557 betrifft eine Synthese von hANP (5 - 28). WO-A-85/04870 offenbart ein Peptid ANP (102 - 126).

Die EP-A-140 731 betrifft Fragmente des ANP, die aus der Ratte isoliert wurden.

WO-A-88/06596 betrifft die Synthese von Urodilatin mittels Festphasensynthese nach Merrifield mit einer Reinheit von mindestens 98 %.

Pept., Proc. Eur. Pept. Symp. 19^{th}, 1987, pp.: 239-42 offenbart ein Puffersystem für eine Umkehrphasen-HPLC-Säule enthaltend Ammoniumphospat, Heptansulfonat und Acetonitrilgradienten.

Aufgabe der Erfindung war es deshalb, ein verbessertes Verfahren zur chemischen Synthese von Cardiodilatin-Fragmenten zu entwickeln, das die oben genannten Nachteile nicht besitzt.

Diese Aufgabe der Erfindung wird gelöst, indem die Synthese von Cardiodilatin-Fragmenten basierend auf dem Merrifield-Verfahren über eine spezielle Auswahl von Peptidfragmenten durchgeführt wird.

Überraschenderweise wurde gefunden, daß die Synthese optimal verläuft, wenn die Cardiodilatin-Fragmente aus drei Teilfragmenten aufgebaut werden, wobei die Kondensation der Teilfragmente zum Cardiodilatin-Fragment der Formel I derart durchgeführt wird, daß der Aufbau über eine Kondensation von Teilftagmenten und Knüpfung der Bindung zwischen der Aminosäureposition Gly¹⁰⁸ und Arg¹⁰⁹ und der Aminosäureposition Gly¹²⁰ und Cys¹²¹ erfolgt. Dieses Verfahren besitzt den Vorteil, daß die Cardiodilatin-Fragmente der Formel I in höheren Ausbeuten und in größerer Reinheit im Vergleich zu dem aus den Stand der Technik bekannten Syntheseverfahren erhalten werden können.

Die Synthese der Cardiodilatin-Fragmente der Formel I erfolgt dabei derart, daß nach dem Merrifield-Verfahren zunächst die drei Teilfragmente mit der Sequenz R¹-ANP(105-108), ANP(109-120) und ANP(121)-R² hergestellt werden. Die Kondensation der drei Teilfragmente zu dem Cardiodilatin-Fragment der Formel I erfolgt dann vorzugsweise in zwei Teilschritten, wobei in einem ersten Schritt die Kondensation zwischen den Aminosäurepositionen Gly¹²⁰ und Cys¹²¹ aus den Teilfragmenten ANP(109-120) und Cys¹²¹-R² erfolgt. Dabei entsteht das Zwischenfragment ANP(109-121)-R². In einem darauffolgenden zweiten Schritt erfolgt dann die Kondensation des so erhaltenenen Fragmentes ANP(109-121)-R² mit dem dritten Teilfragment R¹-ANP(105-108), wobei das gewünschte Cardiodilatin-Fragment der Formel I entsteht. Die Ausbeute an Cardiodilatin-Fragmenten nach dem erfindungsgemäßen Verfahren beträgt zwischen 15 und 20 %, bezogen auf die jeweils als Ausgangsprodukt eingesetzte Menge des Cardiodilatin-Teilfragmentes.

Die drei Teilfragmente mit der Sequenz R¹-ANP(105-108), ANP(109-120) und ANP(121)-R² werden nach dem Merrifield-Verfahren hergestellt, wobei die in der Sequenz vorhandenen Aminosäuren mit funktionalen Gruppen {Hydroxy-, Carboxy-, Amino- oder Mercaptogruppen) durch entsprechende Schutzgruppen substituiert sind. Als geeignete Schutzgruppen kommen beispielsweise folgende Gruppen beispielsweise in Frage:
Schutzgruppen für Hydroxygruppen: BOC (tert. Butyloxycarbonyl), tBu (tert. Butylether).
Schutzgruppen für Aminofünktionen: Fmoc (9-Fluorenylmethyloxy-carbonyl), Pbf (2,2,4,6,7-pentamethyldihydrobenzofuran-5-sulfonyl), Pmc (2,2,5,7,8-Pentamethylchroman-6-sulphonyl), TrT (Trityl).
Schutzgruppe für Carboxygruppen: OtBu (tert. Butylester).
Schutzgruppen für Mercaptogruppen: Acm (Acetamidomethyl) oder Trt.
Hierbei sind folgende Schutzgruppen für die folgenden Aminosäuren bevorzugt: tBu für die Aminosäure Thr, Asn, Tyr oder Ser; Pbf oder Pmc für die Aminosäure Arg; Acm für die Aminosäure Cys; OtBu für die Aminosäure Asp; Trt für die Aminosäure Gin, Asn oder Cys; OtBu für die Aminosäure Asp.

Die geschützten Teilfragmente ANP(109-120), R¹-ANP(105-108) und ANP(121)-R² werden auf einem festen Trägermaterial mittels Fmoc-Strategie aufgebaut (B. Riniker et al., Tetrahedron 1993, 49:9307-9320). Als feste Trägermaterialien können alle in der Merrifield-Synthese gewöhnlich verwendeten Materialien dienen. Als Trägermaterial bevorzugt ist Polystyrol, welches als Aminomethyl- oder Benzhydrylaminoverbindung funktionalisiert ist. Die supersäurelabile Bindung der Peptidfragmente mittels des 4-(4-Hydroxymethyl-3-methoxy-phenoxy)-buttersäure-Linkers an das Harz gestattet deren Abspaltung ohne Beeinträchtigung des Seitenkettenschutzes. Die Fragmente werden durch Digerieren mit verschiedenen Lösungsmitteln gereinigt. Die drei Ausgangsfragmente ANP(109-120), R¹-ANP(105-108) und ANP(121)-R² werden mit am C-Terminus endständiger freier Carboxylgruppe so in guter Reinheit erhalten. Die Ausbeute beim Aufbau der Peptide auf dem Trägerharz ist bei jedem einzelnen Schritt bezüglich der Addition einer Aminosäure nahezu quantitativ und beträgt etwa 97 - 99 %.

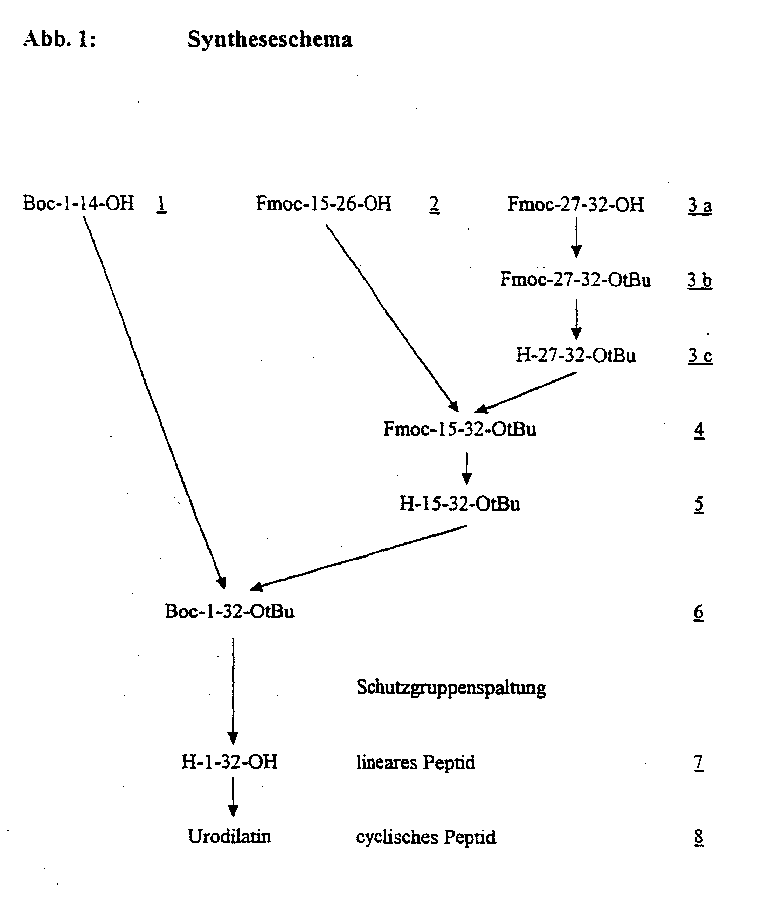

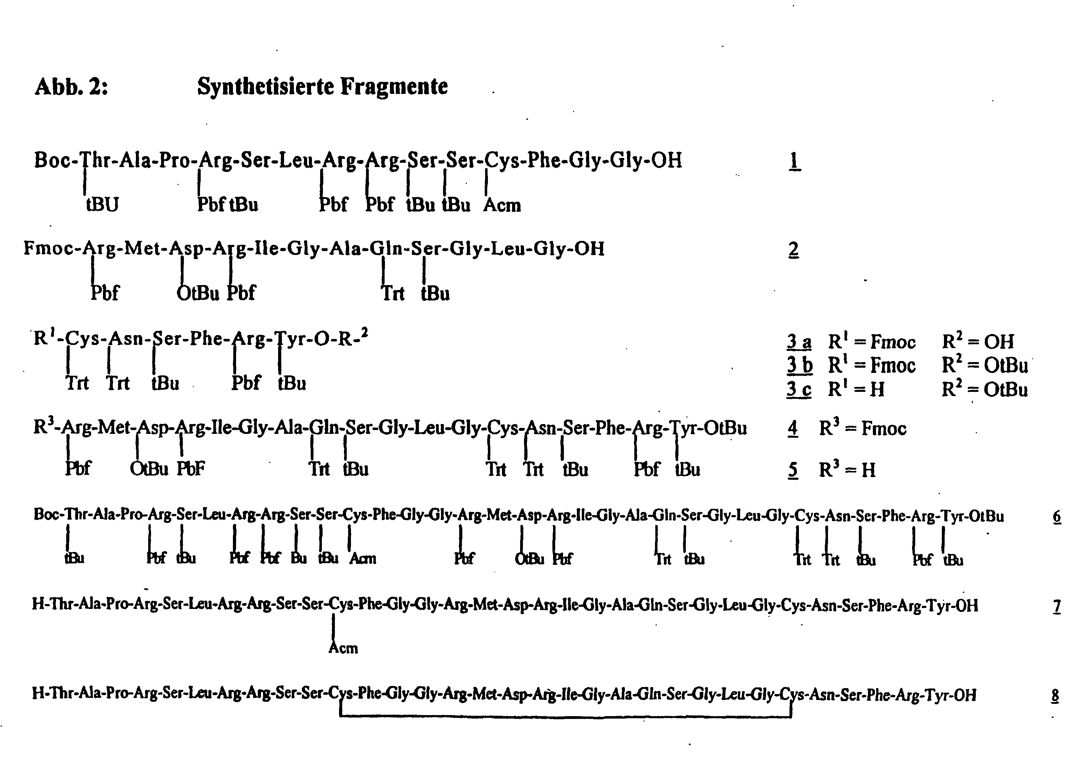

Das Fließschema in Abbildung 1 verdeutlicht das Syntheseprinzip am Beispiel des Urodilatins ANP(95-126). Hierbei erfolgt die Kondensation des Fragments Boc-1-14-OH (1) [diese Nomenklatur entspricht der allgemeinen Bezeichnung des Fragmentes R¹-ANP(105-108) wobei R¹ = ANP(95-104)] mit dem Fragment H-15-32-OtBu (5) [entsprechend der ANP-Nomenklatur ANP(109-121)-R² wobei R² = ANP(122-126)]. Dieses Fragment (5) wird aus den Fragmenten Fmoc-15-26-OH (2) [entsprechend der ANP-Nomenklatur ANP(109-120)] und H-27-32-OtBu (3c) [ensprechend der ANP-Nomenklatur ANP(121)-R²] synthetisiert. Abbildung 2 gibt die synthetisierten und mit Schutzgruppen modifizierten Fragmente wieder.

Im nächsten Schritt wird die Carboxylgruppe des Fragments (3a) in den t-Butylester (3b) überführt (siehe Riniker et al., 22nd Europ. Peptide Symposium, Interlaken, September 1992, (L 7)). Die nachfolgende Abspaltung der Fmoc-Gruppe aus dem Fragment (3b) führt zum Produkt (3c). Dieses wird mit dem Fragment (2) kondensiert und ergibt das Fragment (4). Abspaltung der Fmoc-Schutzgruppe und Kondensation des erhaltenen Fragments (5) mit Fragment (1) führt zum vollgeschützten Urodilatin (6). Die Abspaltung der Schutzgruppen durch Behandlung mit Trifluoressigsäure und 1,3-Propandithiol als Scavenger ergibt das lineare Peptid (7), das durch Oxidation mit Jodlösung zum rohen Urodilatin (8) cyclisiert wird. Dieses wird entsalzt, gereinigt und kann anschließend lyophilisiert werden. Die Synthese anderer Cardiodilatin-Fragmente erfolgt in analoger Weise.

Die erfindungsgemäße Synthese über die beschriebenen Teilfragmente ANP(109-120), R¹-ANP(105-108) und ANP(121)-R² kann für alle Cardiodilatin-Fragmente der Formel I angewandt werden. Insbesondere kommen solche Cardiodilatin-Fragmente in Frage, in denen R¹ eine Kettenlänge von 0-15 Aminosäuren der Sequenz ANP(90-104) oder Fragmente davon aufweist. Bevorzugt sind für R¹ Kettenlängen von 1-15 oder 3-10 Aminosäuren, insbesondere die Sequenzen ANP(95-104), ANP(99-104) und ANP(102-104). Die Gruppe R² steht insbesondere für eine Kettenlänge von 1-5 Aminosäuren der Sequenz ANP(122-126) oder deren Fragmente. Bevorzugt für R² kommen jedoch die Sequenzen ANP(122-126) und ANP(122-125) in Frage.

Vorzugsweise können nach dem erfindungsgemäßen Verfahren die Cardiodilatin-Fragmente ANP(95-126), ANP(99-126) und ANP(102-126) hergestellt werden. Die nach dem erfindungsgemäßen Verfahren hergestellten Cardiodilatin-Fragmente, sowie die für die Kondensation erforderlichen Teilfragmente, besitzen eine sehr hohe optische Reinheit in der Größenordnung von etwa 96 - 99,9 %, insbesondere von etwa 98- 99 %.

Die Synthese eignet sich auch für alle anderen Derivate von Cardiodilatin-Fragmenten, bei denen eine oder mehrere Aminosäuren in der Sequenz des humanen ANP durch andere Aminosäuren ausgetauscht sind. Der Austausch von Aminosäuren in diesem Sinne umfaßt entsprechende Substitutionen, Deletionen oder Insertionen von Aminosäuren. Beispielsweise können einzelne oder mehrere Aminosäuren durch die entsprechenden D-Aminosäuren ausgetauscht werden (vgl. EP 0,180,615). Strukturell ähnliche Peptide mit einer entsprechenden cyclischen Grundstruktur von 15 - 20 Aminosäuren, insbesondere von 17 Aminosäuren, können ebenfalls auf diese Weise hergestellt werden. Beispiele für derartige Peptide sind BNP (brain natriuretic peptide) oder CNP (C-type natriuretic peptide). Die Struktur dieser Peptide ist in J Hypertension 1994, 12: 329-336 (NC Davidson and A.D. Struthers) beschrieben.

Gegenstand der vorliegenden Erfindung sind auch die neuen Teilfragmente des ANP, die zur Herstellung der Cardiodilatin-Fragmente der Formel I nach dem erfindungsgemäßen Verfahren verwendet werden, ANP (95-108), ANP (109-120) sowie ANP (121-126) ErfindungsgemäB einsetzbare Peptidfragmente sind insbesondere solche vom Typ R¹-ANP(105-108), wobei R¹ eine Aminosäurekette der Sequenz ANP(90-104) oder Fragmente davon mit einer Kettenlänge von 0 - 15 Aminosäuren darstellt, sowie deren durch Schutzgruppen modifizierten Derivate. R¹ besitzt dabei insbesondere die zuvor genannten Bedeutungen. Ein weiteres neues Peptidfragment ist das Fragment mit der Aminosäuresequenz ANP(109-120), sowie dessen durch Schutzgruppen modifizierten Derivate, das als Ausgangsprodukt für die Kondensation mit dem Teilfragment ANP(121)-R² eingesetzt wird. Einsetzbar sind auch Peptidfragmente vom Typ AN (121)-R², wobei R² eine Aminosäurenkette der Sequenz ANP(122-126) oder Fragmente davon mit einer Kettenlänge von 0 - 5 Aminosäuren darstellt, sowie deren durch Schutzgruppen modifizierten Derivate. R² hat dabei insbesondere die zuvor genannte Bedeutung. Ebenfalls verwendbar ist das Zwischenprodukt ANP(109-121)-R², das aus der im ersten Reaktionsschritt erfolgten Kondensationsreaktion der Teilfragmente ANP(109-120) und ANP(121-R² entsteht.

Die vorliegende Erfindung betrifft ferner ein Verfahren zur Herstellung von hochreinen Cardiodilatin-Fragmenten der Formel I Die herkömmlichen Syntheseverfahren und anschließenden Reinigungsverfahren von Cardiodilatin-Fragmenten hatten den Nachteil, daß in vielen Fällen lediglich eine Reinheit des Peptids in einer Größenordnung von 97 - 98 % erzielt werden konnte.

EP 0,349,545 beschreibt im Falle des Urodilatins einen Reinheitsgrad von etwa 98 %, wobei die dort hergestellte Menge an Urodilatin nur in kleinerem Labormaßstab in der Größenordnung von wenigen mg erfolgt ist. Das dort in Beispiel 5 beschriebene Reinigungsverfahren basiert auf einer Chromatographie auf einer LH-Säule (Elutionsmittel: 1% AcOH/1% TFEtOH) und anschließender Chromatographie auf einer TSK-Säule (Fractogel TSK-HW 40), wobei als Elutionsmittel eine wässrige Lösung von 10% AcOH und 1% TFEtOH verwendet wurde. In einem letzten Reinigungsschritt erfolgt die Aufreinigung mittels präparativer HPLC ohne weitere Angaben über das Elutionsmittel. Im Rahmen von späteren Versuchen zur Herstellung von größeren Mengen von Urodilatin im Mengenbereich von einigen Gramm zur Durchführung von klinischen Versuchen wurde jedoch festgestellt, daß das synthetisierte Material trotz mehrfach durchgeführter Reinigungsschritte nicht über einen Reinheitsgrad von mehr als 98 % aufgereinigt werden konnte.

Eine vergleichbare Situation ergab sich im Fall der Cardiodilatin-Fragmente, die in EP 0,180,615 beschrieben sind. Dort wird beispielsweise für das Fragment ANP(102-126), das in Beispiel III.A.3 als hANVP(127-151) bezeichnet wird, die Reinigung durch Chromatographie über eine Sephadex-Säule vom Typ G25F beschrieben, wobei als Elutionsmittel 0,5 M AcOH verwendet wurde. In einem anschließenden Reinigungsschritt mittels Ionenaustauschchromatographie an CM-Sepharose oder CM-Cellulose unter Verwendung eines Lösungsmittelgradienten von 0,01 M NH₄OAc / 300 mM NH₄OAc bei ph 4,5 wird das Peptid in einer Reinheit von etwa 97 % erhalten. Auch diese erzielte Reinheit ist nicht zufriedenstellend für die Anforderungen zur Herstellung eines Arzneimittels.

Überraschenderweise wurde gefunden, daß hochreine Cardiodilatin-Fragmente der Formel I hergestellt werden können, wenn die Reinigung des Rohproduktes mittels einer Umkehrphasen-HPLC-Säule erfolgt und das Cardiodilatin-Fragment mit einem Puffersystem enthaltend Triethylammoniumphosphat (TEAP) und Acetonitril in wässriger Lösung eluiert wird. Hierbei wird vorzugsweise der pH-Wert des Elutionspuffers auf einen Wert von 2-5, insbesondere von 2-3 eingestellt. Als Umkehrphasen-HPLC-Säule wird vorzugsweise eine Säule vom Typ C₁₈, beispielsweise vom Typ Biotage-Modul gefüllt mit YMC C₁₈, verwendet. Diese Säule wird vor dem Auftragen der zu reinigenden Cardiodilatin-Fragmente mit einem Triethylammoniumphosphat-Puffer equilibriert. Als geeignete Pufferlösung wird beispielsweise eine Lösung von 10 - 200 mM TEAP, vorzugsweise 50 mM TEAP, eingesetzt. Die Menge an Puffer zum Equilibrieren der Säule ist abhängig von der Größe der Säule und diese wiederum von der Menge des zu reinigenden Peptids. Erfahrungsgemäß wird zur Reinigung einer Peptidmenge von 3-8 g Rohpeptid eine Säulenvolumen von 75 x 300 mm (Durchmesser x Länge) benötigt. In diesem Fall werden etwa 300 ml einer 50 mM TEAP-Pufferlösung zum Equilibrieren benötigt. Anschließend wird eine Lösung des aufkonzentrierten Rohproduktes des Cardiodilatin-Fragmentes aufgetragen. Als Lösungsmittel eignet sich beispielsweise 10 %-ige Essigsäure. Danach wird durch kontinuierliches Zudosieren des Elutionsmittels (Mischung einer wässrigen Lösung von 10 -200 mM TEAP und Acetonitril im Volumenverhältnis 2:3; pH 2-5) das Peptid in einem kontinuierlichen Gradienten eluiert. Besonders vorteilhaft erfolgt die Elution des Peptids, wenn ein kontinuierlicher Gradient des Elutionsmittels angelegt wird, wobei 22 - 28 % des Lösungsmittelsgradienten für eine Dauer von 90 Minuten verwendet wird, anschließend 28 % des Lösungsmittelgradienten während 10 Minuten und schließlich 28 - 40 % des Gradienten in 20 Minuten. Die Flußrate beträgt vorzugsweise 100 - 200 ml/Min., insbesondere etwa 140 ml/Minute. Im Sinne des erfindungsgemäßen Reinigungsverfahrens wird als Elutionspuffer eine Puffermischung von Triethylammoniumphosphat in Wasser und Acetonitril in einem Mischungsverhältnis von 1:3 bis 2:1 (v/v), insbesondere von etwa 2:3 (v/v) verwendet. Der pH-Wert der Pufferlösung beträgt 2-5, vorzugsweise 2-3, und insbesondere etwa 2,25. TEAP kann in einer Konzentration von 10 - 200 mM, bevorzugt 20 - 100 mM und insbesondere von etwa 50 mM eingesetzt werden. Eine optimale Trennung wird erfindungsgemäß in der Umkehrphasen-HLPC durch Equilibrieren der Säule mit 50 mM TEAP pH 2,25 und Eluieren des Peptides mit einem Puffer bestehend aus 50 mM TEAP pH 2,25 und Acetonitril im Verhältnis 2:3 erreicht.

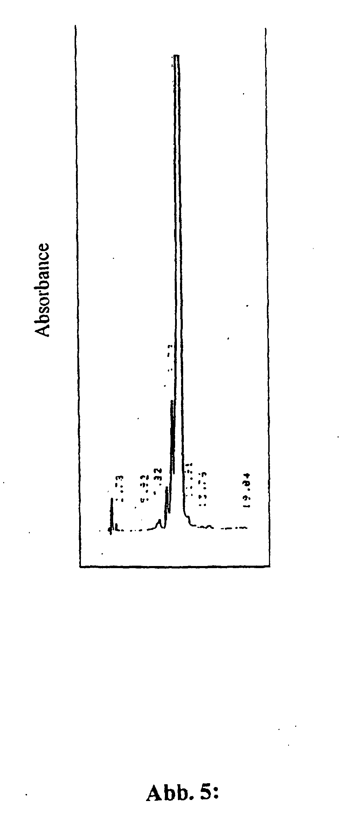

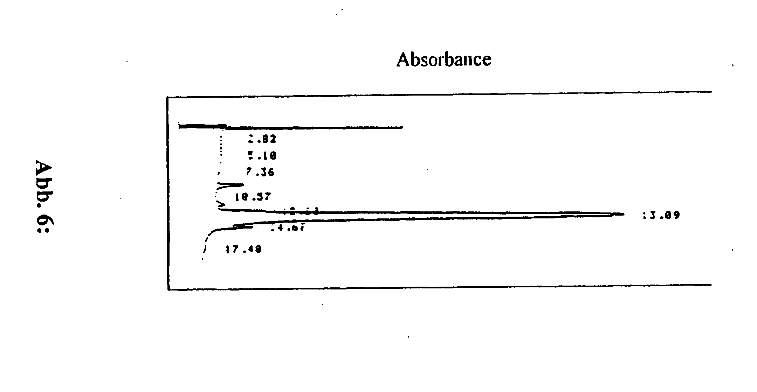

Die herkömmlichen Reinigungsverfahren unter Verwendung von beispielsweise wässriger 0,1 %-iger Trifluoressigsäure (TFA) können die in den Rohprodukten enthaltenen polaren Verunreinigungen, wie sie in Abb. 5 anhand des Beispiels für Urodilatin ersichtlich sind, nicht weiter auftrennen (s. Abb. 6). Im Falle der erfindungsgemäß verwendeten Elutionsmittel hingegen findet eine deutliche Abtrennung der beiden Verunreinigungen statt (s. Abb. 5). Die Verwendung des erfindungsgemäßen Elutionsmittels hat ferner den Vorteil, daß die Basislinie im HPLC-Chromatogramm absolut ruhig verläuft, während im Falle von TFA ein starker Drift zu beobachten ist. Außerdem hat die Verwendung von TFA den Nachteil, daß ein höherer Gegendruck auf der HPLC-Säule aufgebaut wird, was im Falle des erfindungsgemäßen Elutionsmittels nicht der Fall ist.

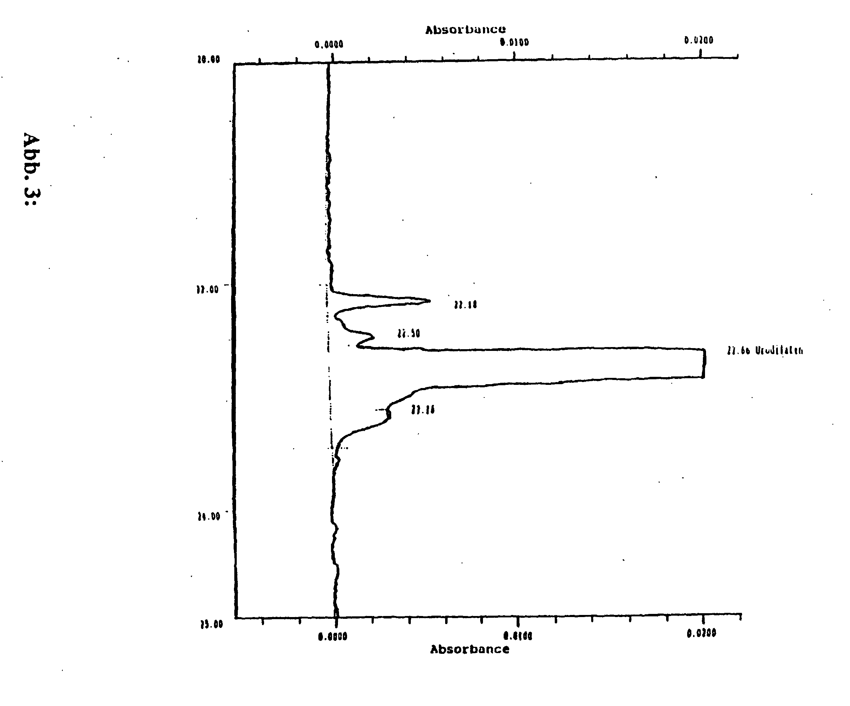

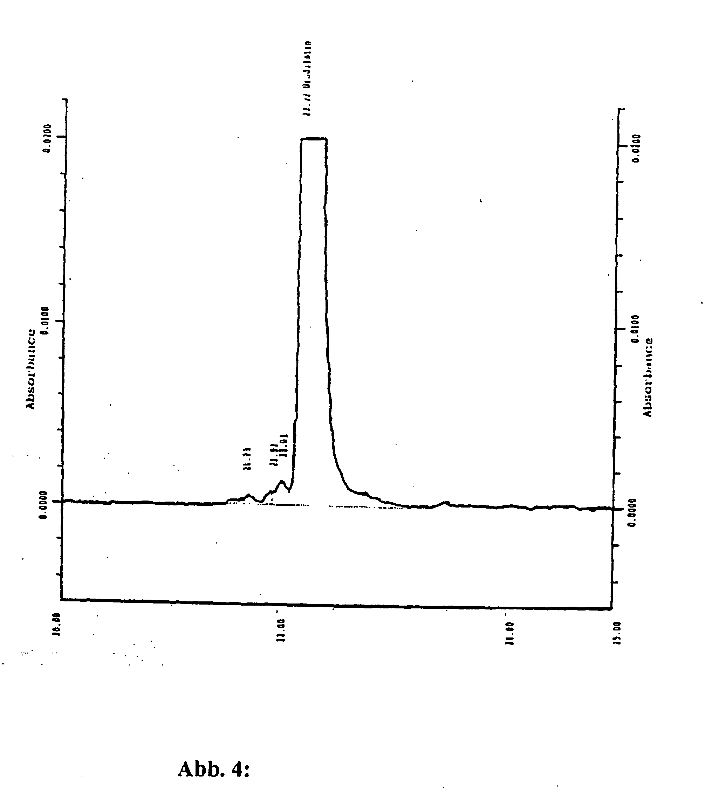

Mit dem erfindungsgemäßen Verfahren werden hochreine Cardiodilatin-Fragmente der Formel I mit einer Reinheit von mindestens 99 %, und vorzugsweise bis zu ungefähr 99,9 % erhalten. Die Cardiodilatin-Fragmente können gegebenenfalls anschließend in ihre physiologisch verträgliche Salze, wie z.B. Acetat- oder Citratsalz, überführt werden. Die erhaltenen Cardiodilatin-Fragmente sind im wesentlichen frei von Peptidverunreinigungen, so daß nicht nur die Umkehrphasen-HPLC einen Einzelpeak aufweist, sondern auch die weit empfindlichere Methode der Kapillarelektrophorese (CE) einen Einzel-Migrationspeak ergibt. Er zeigt im Falle des Urodilatins in der MS-Analyse eine Masse von 3505,9 +/-1 ohne Detektion von Nebenprodukten. Es zeigte sich, daß es mittels Kapillarelektrophorese hervorragend möglich ist, die Unterschiede zwischen dem nach dem Stand der Technik erhaltenen Cardiodilatin-Fragmenten und den erfindungsgemäßen Cardiodilatin-Fragmenten deutlich zu machen. Abbildung 3 gibt das CE-Chromatogramm einer nach dem Stand der Technik hergestellten Produktionscharge von Urodilatin an. Hierbei ist deutlich zu erkennen, daß noch Verunreinigungen im Produkt enthalten sind. Abbildung 4 hingegen stellt das CE-Chromatogramm einer nach dem erfindungsgemäßen Verfahren hergestellten und entsprechend gereinigten Produktionscharge von Urodilatin dar. Es ist deutlich zu erkennen, daß das Produkt im wesentlichen frei von anderen Peptidverunreinigungen ist und in der Kapillarelektrophorese einen Einzel-Migrationspeak aufweist.

Gegenstand der Erfindung sind deshalb hochreine Cardiodilatin-Fragmente der Formel I, die sich dadurch auszeichnen, daß sie im wesentlichen keine durch Kapillarelektrophorese und MS-Analyse nachweisbaren Peptidverunreinigungen enthalten und die Reinheitsanalyse mittels Kapillarelektrophorese im wesentlichen einen Einzel-Migrationspeak aufweist.

Das erfindungsgemäße Reinigungsverfahren eignet sich ebenfalls zur Herstellung von hochreinen analogen Peptidverbindungen, die eine vergleichbare cyclische Struktur von wie ANP besitzen, wie zum Beispiel BNP (brain natriuretic peptide), CNP (C-type natriuretic peptide) und deren Derivate. Die cyclische Struktur von ANP beruht auf der Oxidation von zwei Cystein-Resten in der Aminosäuresequenz, wobei sich ein cyclischer Ring von 17 Aminosäuren bildet. Andere Peptide, die ebenfalls die charakteristische cyclische Struktur von 15 - 20 Aminosäuren, insbesondere von 17 Aminosäuren ausbilden, wie z.B. BNP oder CNP, können auf die gleiche Weise nach dem erfindungsgemäßen Reinigungsverfahren in die hochgereinigten Formen überführt werden.

In den folgenden Ausführungsbeispielen wird die Erfindung anhand der repräsentativ ausgewählten Cardiodilatin-Fragmenten ANP(95-126), ANP(99-126) und ANP(102-126) verdeutlicht.

### Beispiel 1:

### Allgemeine Vorschriften zur Festphasensynthese nach dem Merrifield-Verfahren

### a) Festphasensynthese am Trägerharz

Ausgehend vom C-terminalen Ende des zu synthetisierenden Peptids wird die erste Aminosäure (AA), die am N-terminalen Ende durch die Fmoc-Gruppe geschützt ist, an das Trägerharz gebunden (Fmoc-AA-OHMPB-Trägerharz). Bei einem Standardansatz von 6,66 mMol erfolgt anschließend die Abspaltung der Fmoc-Schutzgruppe durch Zugabe von 100 ml eines Lösungsmittelgemisches von Piperidin und N-Methylpyrrolidin (1:4 v/v). Die Suspension des Harzes wird dann 10 Minuten gerührt, anschließend filtriert und nochmals 100 ml des Lösungsmittelgemisches von Piperidin und NMP zugegeben. Die Suspension wird dann 10 Minuten gerührt, filtriert und anschließend mit NMP und Isopropanol gewaschen und die Vollständigkeit der Reaktion mit Hilfe des Kaisertests überprüft.

Danach erfolgt die Kupplung der nächsten Aminosäure an das Harz. Zunächst werden 20 mMol einer 0,5 M Lösung von Düsoproplyethanylamin (DIPEA) in NMP zum Harz zugegeben, dann 2,5 mMol einer 0,5 M Lösung von 1-Hydroxybenzotriazol (HOBT) in NMP und anschließend 10 mMol der zu koppelnden Aminosäure in 25 ml NMP. Dann werden noch 11 mMol einer 0,25 M Lösung von TBTU (2-(1H-Benzotriazol-1-yl)-1,1,3,3-tetramethyluronium-tetrafluorborat) in NMP zugegeben und 10 Minuten gerührt. Die Vollständigkeit der Reaktion wird mit Hilfe des Kaisertests überprüft. Anschließend wird das Harz filtriert und mit NMP gewaschen.

Auf diesselbe Weise wird weiter verfahren, bis die Peptidkette mit der gewünschten Kettenlänge von Aminosäuren auf dem Harz aufgebaut ist. Am Schluß der Synthese wird das Harz bis zur Gewichtskonstanz bei 40 °C getrocknet.

### b) Abspaltung der geschützten Peptide vom Trägerharz

In 10 Saugflaschen werden je 75 ml Methanol und 3 ml Pyridin vorgelegt. 50 g des nach Schritt a) hergestellten Peptidharzes werden 10 mal mit je 250 ml 1 % TFA in trockenem Methylenchlorid während je einer Minute auf der Nutsche gerührt und direkt in die jeweilige Saugflasche filtriert. Die 10 Filtrate werden dünnschichtchromatographisch untersucht. Produkthaltige Fraktionen werden vereint und zur Trockene verdampft. Der Rückstand wird mit entionisiertem Wasser verrieben und der kristalline Rückstand abfiltriert und getrocknet.

### Beispiel 2:

### Herstellung des Fragmentes ANP(109-120)

Ausgehend von 273 g Fmoc-Gly-OHMPB-Trägerharz (entspricht 130 mMol) werden nach den allgemeinen Vorschriften nach Beispiel 1 170,3 g des vollgeschützten Cardiodilatin-Fragmentes ANP(109-120) erhalten.

### Beispiel 3:

### Herstellung des Fragmentes ANP(121-126)

Ausgehend von 264 g Fmoc-Tyr-OHMPB-Trägerharz (entspricht 115 mMol) werden nach den allgemeinen Vorschriften nach Beispiel 1 150,7 g des vollgeschützten Cardiodilatin-Fragmentes ANP(121-126) erhalten. Das N-terminale Ende des Fragmentes ist hierbei durch die Fmoc-Gruppe geschützt.

Die endständige Hydroxygruppe am C-terminalen Ende des Fragementes wird anschlieβend in die otbu-Schutzgruppe überführt. Zur Veresterung werden 149 g des vollgeschützten Fragmentes in 500 ml Trifluorethanol und 4,1 l Chloroform gelöst. Anschließend werden 141 ml TBTA (tert.Butyl-2,2,2-trichloracetimidat) zugegeben und die Lösung eine Stunde unter Rückfluß erwärmt. Nach vollständiger Reaktion wird die Lösung zum kristallin-öligen Rückstand eingeengt, 6,8 l Düsopropylether zugegeben und die Suspension 14 h bei Raumtemperatur gerührt. Das Produkt wird abfiltriert und bis zur Gewichtskonstanz getrocknet. Es werden 136,7 g des in Abb. 2 angegebenen Fragmentes 3b erhalten.

Anschließend erfolgt die Abspaltung der Fmoc-Schutzgruppe am N-terminalen Ende des Fragmentes und die Überführung in das in Abb. 2 angegebene Fragment 3c. Hierzu wird eine Lösung von Fragment 3b (135,7 g) in 1,8 l DMF und 74 ml Diethylamin bei Raumtemperatur drei Stunden lang gerührt. Die Lösung wird im Vakuum vollständig zur Trockne eingedampft. Der Rückstand wird mit 1,4l entionisiertem Wasser digeriert und abfiltriert. Das noch feuchte Produkt wird in 3 l MTBE (Methyl-tert.-Butylether) aufgenommen. Die Lösung wird mit einer gesättigten NaCl-Lösung (2 x 100 ml) extrahiert und die organische Phase mit Natriumsulfat getrocknet. Die Lösung wird auf ein Volumen von 500 ml eingeengt. Nach Zugabe von 1,5l Isopropylether wird für zwei Stunden gerührt. Das Produkt wird filtriert und getrocknet. Die Ausbeute beträgt 104,6 g des in Abb. 2 angegebenen Fragmentes 3c.

### Beispiel 4:

### Herstellung des Fragmentes ANP(121-125)

In analoger Weise wie in Beispiel 3 beschrieben werden ausgehend von 264 g Fmoc-Arg(Pbf)-OHMPB-Trägerharz nach dem beschriebenen Verfahren 115,1 g des Cardiodilatin-Fragmentes ANP(121-125) erhalten.

### Beispiel 5:

### Herstellung des Fragmentes ANP(95-108)

Ausgehend von 210 g Fmoc-CHy-OHMPB-Trägerharz werden nach den allgemeinen Vorschriften nach Beispiel 1 151,5 g des vollgeschützten Cardiodilatin-Fragmentes ANP(95-108) erhalten.

### Beispiel 6:

### Herstellung des Fragmentes ANP(99-108)

Ausgehend von 190 g Fmoc-Gly-OHMPB-Trägerharz werden nach den allgemeinen Vorschriften nach Beispiel 1 145,1 g des vollgeschützten Cardiodilatin-Fragmentes ANP(99-108) erhalten.

### Beispiel 7:

### Herstellung des Fragmentes ANP(102-108)

Ausgehend von 220 g Fmoc-Gly-OHMPB-Trägerharz werden nach den allgemeinen Vorschriften nach Beispiel 1 165,3 g des vollgeschützten Cardiodilatin-Fragmentes ANP(102-108) erhalten.

### Beispiel 8:

### Kondensation der Teilfragmente zum Zwischenprodukt

Das Fragment ANP(109-120) wird durch Kondensation mit dem C-terminalen Fragment ANP(121)-R² zum Zwischenprodukt ANP(109-121)-R² nach dem folgenden allgemeinen Verfahren umgesetzt:

Das Fragment ANP(109-120), dessen Aminoterminus durch die Fmoc-Gruppe geschützt ist, wird in N-Methylpyrrolidon gelöst. Anschließend wird die Lösung bei Zimmertemperatur mit TBTU (2-(1H-Benzotriazol-1-yl). 1,1,3,3-tetramethyluronium-tetralluorborat), 1-Hydroxybenzotriazol und Diisopropylethylamin unter Rühren versetzt. Danach wird die Lösung mit dem am C-terminalen Ende durch eine entsprechende Schutzgruppe versehenen Fragment ANP(121)-R², gelöst in N-Methylpyrrolidon, versetzt. Die Reaktion wird anschließend dünnschichtchromatographisch verfolgt. Nach ca. 2 Stunden ist die Umsetzung abgeschlossen. Das Reaktionsgemisch wird dann unter Rühren aufDiisopropylether getropft und anschließend ca. 30 Minuten gerührt. Der ausgefallene Niederschlag wird auf einer Porzellannutsche über Hartfilter filtriert und zweimal mit Düsopropylether gewaschen. Der Rückstand wird anschließend in Acetonitril suspendiert und unter Rühren bei Zimmertemperatur digeriert. Anschließend wird das Produkt auf einer Porzellannutsche abfiltriert, nochmals mit Acetonitril gewaschen und im Vakuumschrank bis zur Gewichtskonstanz bei 40 °C getrocknet. Das so erhaltene Rohprodukt stellt das am Aminoterminus durch die Schutzgruppe Fmoc geschützte Cardiodilatin-Fragment Fmoc-ANP(109-121)-R² dar. Anschließend wird die Fmoc-Gruppe nach bekannten Verfahren abgespalten, so daß das Zwischenprodukt H-ANP(109-121)-R² erhalten wird.

### Beispiel 9:

### Kondensation der Fragmente ANP(109-120) mit ANP(121-126) zu ANP(109-126)

Nach dem in Beispiel 8 beschriebenen allgemeinen Verfahren werden 21,6 g Fmoc-ANP(109-120) in 650 ml N-Methylpyrrolidon gelöst. Anschließend wird die Lösung bei Zimmertemperatur mit 3,2 g TBTU (2-(1H-Benzotriazol-l-yl).1,1,3,3-tetramethyluronium-tetrafluorborat), 1,5 g 1-Hydroxybenzotriazol und 3,5 ml Düsopropylethylamin unter Rühren versetzt. Danach wird eine Lösung von H-ANP(121-126)-OtBu gelöst in 150 ml N-Methylpyrrolidon zugegeben. Die Reaktion wird anschließend dünnschichtchromatographisch verfolgt. Nach ca. 2 Stunden ist die Umsetzung abgeschlossen. Das Reaktionsgemisch wird dann unter Rühren auf 4 1 Diisopropylether getropft und anschließend ca. 30 Minuten gerührt. Der ausgefallene Niederschlag wird auf einer Porzellannutsche über Hartfilter filtriert und zweimal mit 500 ml Diisopropylether gewaschen. Der Rückstand wird anschließend in 600 ml Acetonitril suspendiert und unter Rühren bei Zimmertemperatur digeriert. Anschließend wird das Produkt auf einer Porzellannutsche abfiltriert, nochmals mit 300 ml Acetonitril gewaschen und im Vakuumschrank bis zur Gewichtskonstanz bei 40 °C getrocknet. Das so in einer Menge von 32,3 g erhaltene Rohprodukt Fmoc-ANP(109-126) wird anschließend durch Zusatz von Diethylamin in das ungeschützte ANP(109-126) überführt. Die Ausbeute beträgt 30,2 g.

### Beispiel 10:

### Kondensation der Fragmente ANP(109-120) mit ANP(121-125) zu ANP(109-125)

Nach dem in Beispiel 8 beschriebenen allgemeinen Verfahren werden 18,6 g Fmco-ANP(109-120) in 600 ml N-Methylpyrrolidon gelöst. Anschließend wird die Lösung bei Zimmertemperatur mit 3,0 g TBTU (2-(1H-Benzotriazol-1-yl).1,1,3,3-tetramethyluronium-tetrafluorborat), 1,2 g 1-Hydroxybenzotriazol und 3,0 ml Diisopropylethylamin unter Rühren versetzt. Danach wird eine Lösung von H-ANP(121-125)-OtBu gelöst in 150 ml N-Methylpyrrolidon zugegeben. Die Reaktion wird anschließend dünnschichtchromatographisch verfolgt. Nach ca. 2 Stunden ist die Umsetzung abgeschlossen. Das Reaktionsgemisch wird dann unter Rühren auf 4l Diisopropylether getropft und anschließend ca. 30 Minuten gerührt. Der ausgefallene Niederschlag wird auf einer Porzellannutsche über Hartfilter filtriert und zweimal mit 450 ml Diisopropylether gewaschen. Der Rückstand wird anschließend in 500 ml Acetonitril suspendiert und unter Rühren bei Zimmertemperatur digeriert. Anschließend wird das Produkt auf einer Porzellannutsche abfiltriert, nochmals mit 250 ml Acetonitril gewaschen und im Vakuumschrank bis zur Gewichtskonstanz bei 40 °C getrocknet. Das so in einer Menge von 29,1 g erhaltene Rohprodukt Fmoc-ANP(109-125) wird anschließend durch Zusatz von Diethylamin in das ungeschützte ANP(109-125) überführt. Die Ausbeute beträgt 28,2 g.

### Beispiel 11:

### Kondensation der Teilfragmente zum Endprodukt

Das Zwischenprodukt ANP(109-121)R² wird durch Kondensation mit dem Amino-tenninalen Fragment R¹-ANP(105-108) zum Endprodukt R¹-ANP(105-121)-R² nach dem folgenden allgemeinen Verfahren umgesetzt:

Das Fragment R¹-ANP(105-108), dessen Aminoterminus durch eine entsprechende Schutzgruppe geschützt ist, wird in N-Methylpyrrolidon gelöst. Anschließend wird die Lösung bei Zimmertemperatur mit TBTU (2-{1H-Benzotriazol-1-yl).1,1,3,3-tetramethyluronium-tetrafluorborat), 1-Hydroxybenzotriazol und Düsopropylethylamin unter Rühren versetzt. Danach wird die Lösung mit dem am C-terminalen Ende durch eine entsprechende Schutzgruppe versehenen Fragment ANP(109-121)-R², gelöst in N-Methylpyrrolidon, versetzt. Die Reaktion wird anschließend dünnschichtchromatographisch verfolgt. Nach ca. 2 Stunden ist die Umsetzung abgeschlossen. Das Reaktionsgemisch wird dann unter Rühren auf Diisopropylether getropft und anschließend ca. 30 Minuten gerührt. Der ausgefallene Niederschlag wird auf einer Porzellannutsche über Hartfilter filtriert und zweimal mit Düsopropyiether gewaschen. Der Rückstand wird anschließend in Acetonitirl suspendiert und unter Rühren bei Zimmertemperatur digeriert. Anschlieβend wird das Produkt auf einer Porzellannutsche abfiltriert, nochmals mit Acetonitril gewaschen und im Vakuumschrank bis zur Gewichtskonstanz bei 40 °C getrocknet. Das so erhaltene Rohprodukt stellt das am Aminoterminus und am C-Terminus durch entsprechende Schutzgruppen geschützte Cardiodilatin-Fragment R¹-ANP(105-121)-R² dar. Anschließend wird die Schutzgruppe nach bekannten Verfahren abgespalten, so daß das Zwischenprodukt H-R¹-ANP(109-121)-R² erhalten wird. Nach dem vollständigen Entfernen der Schutzgruppen wird nach bekannten Verfahren das erhaltene Cardiodilatin-Fragment durch Oxidation, beispielsweise mit Jod, in das cyclisierte Derivat überführt.

### Beispiel 12:

### Kondensation der Fragmente ANP(109-126) und ANP(95-108) zu ANP(95-126)

### a) Herstellung von ANP(95-126)

Nach dem in Beispiel 11 beschriebenen allgemeinen Verfahren werden 20,6 g Boc-ANP(95-108) in 400 ml N-Methylpyrrolidon gelöst. Anschließend wird die Lösung bei Zimmertemperatur mit 2,7 g TBTU (2-(1H-Benzotriazol-l-yl).1,1,3,3-tetramethyluronium-tetrafluorborat), 1,3 g 1-Hydroxybenzotriazol und 2,7 ml Diisopropylethylamin unter Rühren versetzt. Danach wird eine Lösung von 29,4 g H-ANP(109-126)-OtBu gelöst in 400 ml N-Methylpyrrolidon zugegeben. Die Reaktion wird anschließend dünnschichtchromatographisch verfolgt. Nach ca. 2 Stunden ist die Umsetzung abgeschlossen. Das Reaktionsgemisch wird dann unter Rühren auf 6,5 l Diisopropylether getropft und anschließend ca. 30 Minuten gerührt. Der ausgefallene Niederschlag wird auf einer Porzellannutsche über Hartfilter filtriert und zweimal mit 500 ml Diisopropylether gewaschen. Der Rückstand wird anschließend in 600 ml Acetonitril suspendiert und unter Rühren bei Zimmertemperatur digeriert. Anschließend wird das Produkt auf einer Porzellannutsche abfiltriert, nochmals mit 500 ml Acetonitril gewaschen und im Vakuumschrank bis zur Gewichtskonstanz bei 40 °C getrocknet. Das so in einer Menge von 42,5 g erhaltene Rohprodukt Boc-ANP(95-126)-OtBu wird anschließend in das ungeschützte ANP(95-126) überführt und getrocknet. Die Ausbeute beträgt 27,5 g.

### b) Cyclisation des entschützten, linearen ANP(95-126)

60 g des ungeschützten ANP(95-126) werden in 16 l 5 % Essigsäure in deionisiertem Wasser (v/v) gelöst und durch Zugabe von 570 ml einer 0,02 M methanolischen Jodlösung oxidiert. Die Reaktion ist nach 5 Minuten beendet. Das überschüssige Jod wird durch Zugabe einer 0,1 M Natriumthiosulfat-Lösung vernichtet. Die erhaltene Cyclisationslösung wird direkt weiter verarbeitet.

### Beispiel 13:

### Kondensation der Fragmente ANP(109-126) und ANP(99-108) zu ANP(99-126)

Analog zu dem in Beispiel 12 beschriebenen Verfahren werden 22,5 g Boc-ANP(99-108) in 400 ml N-Methylpyrrolidon gelöst. Anschließend wird die Lösung bei Zimmertemperatur mit 2,9 g TBTU (2-(1H-Benzotriazol-1-yl).1,1,3,3-tetramethyluronium-tetrafluorborat), 1,4 g 1-Hydroxybenzotriazol und 2,8 ml Diisopropylethylamin unter Rühren versetzt. Danach wird eine Lösung von 30,6 g H-ANP(109-126)-OtBu gelöst in 400 ml N-Methylpyrrolidon zugegeben. Die Reaktion wird anschließend dünnschichtchromatographisch verfolgt. Nach ca. 2 Stunden ist die Umsetzung abgeschlossen. Das Reaktionsgemisch wird dann unter Rühren auf 6,5 l Diisopropylether getropft und anschließend ca. 30 Minuten gerührt. Der ausgefallene Niederschlag wird auf einer Porzellannutsche über Hartfilter filtriert und zweimal mit 500 ml Diisopropylether gewaschen. Der Rückstand wird anschließend in 600 ml Acetonitril suspendiert und unter Rühren bei Zimmertemperatur digeriert. Anschließend wird das Produkt auf einer Porzellannutsche abfiltriert, nochmals mit 500 ml Acetonitril gewaschen und im Vakuumschrank bis zur Gewichtskonstanz bei 40 °C getrocknet. Das so in einer Menge von 44,7 g erhaltene Rohprodukt Boc-ANP(99-126)-OtBu wird anschließend in das ungeschützte ANP(99-126) überführt und getrocknet. Die Ausbeute beträgt 28,1 g.

### Beispiel 14:

### Kondensation der Fragmente ANP(109-126) und ANP(102-108) zu ANP(102-126)

Analog zu dem in Beispiel 12 beschriebenen Verfahren werden 20,4 g Boc-ANP(102-108) in 360 ml N-Methylpyrrolidon gelöst. Anschließend wird die Lösung bei Zimmertemperatur mit 2,7 g TBTU (2-(1H-Benzotriazol-1-yl).1,1,3,3-tetramethyluronium-tetrafluorborat), 1,4 g 1-Hydroxybenzotriazol und 2,6 ml Diisopropylethylamin unter Rühren versetzt. Danach wird eine Lösung von 30,1 g H-ANP(109-126)-OtBu gelöst in 400 ml N-Methylpyrrolidon zugegeben. Die Reaktion wird anschließend dünnschichtchromatographisch verfolgt. Nach ca. 2 Stunden ist die Umsetzung abgeschlossen. Das Reaktionsgemisch wird dann unter Rühren auf 6,5 Diisopropylether getropft und anschließend ca. 30 Minuten gerührt. Der ausgefallene Niederschlag wird auf einer Porzellannutsche über Hartfilter filtriert und zweimal mit 500 ml Diisopropylether gewaschen. Der Rückstand wird anschließend in 600 ml Acetonitril suspendiert und unter Rühren bei Zimmertemperatur digeriert. Anschließend wird das Produkt auf einer Porzellannutsche abfiltriert, nochmals mit 500 ml Acetonitril gewaschen und im Vakuumschrank bis zur Gewichtskonstanz bei 40 °C getrocknet. Das so in einer Menge von 41,2 g erhaltene Rohprodukt Boc-ANP(102-126)-OtBu wird anschließend in das ungeschützte ANP(102-126) überführt und getrocknet. Die Ausbeute beträgt 26,9 g.

### Beispiel 15:

### Reinigung von ANP(95-126) und Herstellung der hochreinen Form

### a) Aufkonzentrierung des cyclisierten Urodilatins [ANP(95-126)]

Die Cyclisationslösung (ca. 17 Liter 5 % AcOH, in deionisiertem Wasser (v/v), enthält ca. 60 g cyclisiertes Urodilatin) wird auf eine mit 1000 ml Puffer A3 (0,1 % TFA (v/v) in deionisiertem Wasser) equilibrierte Glassäule (Durchmesser: 70 mm, Länge: 900 mm, gefüllt mit Vydac 218 TPB 2030) aufgetragen (Flußrate 130 ml/Min.).

Nach beendetem Aufpumpen wird das Peptid durch kontinuierliches Zudosieren von Puffer B3 (0,1 % TFA in deionisiertem Wasser/ACN 2:3 v/v) in einem kontinuierlichen Gradienten eluiert (0 % Puffer B während 40 Min.; 15-35 % Puffer B in 90 Min.; 35 % B während 10 Min.; Flussrate 130 ml/Min.).

Urodilatin-Fraktionen, die beim Monitorieren mittels analytischer HPLC eine Reinheit größer 75% zeigen, werden vereinigt. Diese vereinigten Fraktionen werden mit einem Volumenäquivalent deionisiertem Wasser verdünnt und auf ein mit 300 ml Puffer A3 equilibriertes Biotage-Modul (Durchmesser 75 mm, Länge 300 mm, gefüllt mit YMC C₁₈, 120 A, 10 µm) aufgetragen (Flußrate 140 ml/Min.).

Anschließend wird das konzentrierte Peptid durch Spülen der Säule mit 100% Puffer B3 eluiert und das Acetonitril abgedampft. Die verbleibende Lösung wird lyophilisiert.

Es resultieren zwischen 17 und 20 g Urodilatin mit einer Reinheit größer 90%.

### b) Reinigung des aufkonzentrierten Urodilatins

4,5 g des aufkonzentrierten Urodilatins werden in 250 ml 10 % AcOH, in deionisiertem Wasser (v/v), gelöst und auf ein mit 300 ml Puffer A4 (50 mM TEAP pH 2,25 in deionisiertem Wasser) equilibriertes Biotage-Modul (Durchmesser 75 mm, Länge 300 mm, gefüllt mit YMC C₁₈, 120 A, 10 µm) aufgetragen (Flußrate 140 ml/Min.)

Das Peptid wird durch kontinuierliches Zudosieren von Puffer B4 (50 mM TEAP pH 2,25 in deionisiertem Wasser /ACN 2:3 v/v) in einem kontinuierlichen Gradienten eluiert (22-28 % B in 90 Min.; 28 % B während 10 Min.; 28-40 % B in 20 Min.; Flußrate 140 ml/Min.).

Urodilatin-Fraktionen, die beim Monitorieren mittels analytischer HPLC eine Reinheit größer 99 % und dabei keine Verunreinigung größer 0,5 % zeigen, werden vereinigt. Diese vereinigten Fraktionen werden mit einem Volumenäquivalent deionisiertem Wasser verdünnt und auf das zuvor mit 1000 ml Puffer B3 gereinigte und anschließend mit 300 ml Puffer A3 equilibrierte Biotage-Modul aufgepumpt. Zur Entsalzung wird mit 1200 ml Puffer A3 gespült.

Das Reinprodukt wird durch Spülen der Säule mit 1500 ml Puffer B3 eluiert und das Acetonitril abgedampft. Die verbleibende Lösung wird lyophilisiert.

Es resultieren zwischen 2,3 und 2,7 g hochreines Urodilatin.

### c) Umsalzung von Urodilatin x TFA zu Urodilatin-Acetat

2,5 g hochreines Urodilatin x TFA-Salz werden in 80 ml 5 % AcOH, in deionisiertem Wasser v/v, gelöst und auf eine mit 5% AcOH gespülte Chromatographiesäule (Durchmesser 20 mm, Länge 300 mm, gefüllt mit 40 ml Merck Ionenaustauscher III Acetat-Form) aufgetragen. Es wird mit 40 ml 5 % AcOH gewaschen. Das Eluat, ca. 125 ml, wird noch einmal auf die gleiche Ionenaustauschersäule appliziert. Es wird mit 55 ml % AcOH gewaschen. Das Eluat, ca 180 ml, wird über eine Polysulfonmembran (Durchmesser 47 mm, 0,2 µm) klarfiltriert. Die Lösung wird lyophilisiert.

Es resultieren zwischen 2,05 und 2,30 g hochreines Urodilatin-Acetat.

### Beispiel 16:

### Reinigung von ANP(99-126) und Herstellung der hochreinen Form

### a) Aufkonzentrierung des cyclisierten Cardiodilatin-Fragmentes ANP(99-126)

Analog zu Beispiel 15 a) wird die Cyclisationslösung (ca. 15 Liter 5 % AcOH, in deionisiertem Wasser (v/v), mit einem Peptidgehalt von ca. 50 g) auf eine mit 1000 ml Puffer A3 (0,1 % TFA (v/v) in deionisiertem Wasser) equilibrierte Glassäule aufgetragen (Flußrate 130 ml/Min.). Nach beendetem Aufpumpen wird das Peptid durch kontinuierliches Zudosieren von Puffer B3 (0,1 % TFA in deionisiertem Wasser/ACN 2:3 v/v) in einem kontinuierlichen Gradienten eluiert (0 % Puffer B während 40 Min.; 15-35 % Puffer B in 90 Min.; 35 % B während 10 Min.; Flussrate 130 ml/Min.). Peptid-Fraktionen, die beim Monitorieren mittels analytischer HPLC eine Reinheit größer 75% zeigen, werden vereinigt. Diese vereinigten Fraktionen werden mit einem Volumenäquivalent deionisiertem Wasser verdünnt und auf ein mit 300 ml Puffer A3 equilibriertes Biotage-Modul aufgetragen (Flußrate 140 ml/Min.). Anschließend wird das konzentrierte Peptid durch Spülen der Säule mit 100% Puffer B3 eluiert und das Acetonitril abgedampft. Die verbleibende Lösung wird lyophilisiert.

Es resultieren zwischen 14 und 17 g des Cardiodilatin-Fragmentes ANP(99-126) mit einer Reinheit größer 90% .

### b) Reinigung des aufkonzentrierten ANP(99-126)

3,5 g des nach Beispiel 16 a) aufkonzentrierten Cardiodilatin-Fragmentes werden in 200 ml 10 % AcOH, in deionisiertem Wasser (v/v), gelöst und auf ein mit 300 ml Puffer A4 (50 mM TEAP pH 2,25 in deionisiertem Wasser) equilibriertes Biotage-Modul aufgetragen (Flußrate 140 ml,Min.). Das Peptid wird durch kontinuierliches Zudosieren von Puffer B4 (50 mM TEAP pH 2,25 in deionisiertem Wasser /ACN 2:3 v/v) in einem kontinuierlichen Gradienten eluiert (22-28 % B in 90 Min.; 28 % B während 10 Min.; 28-40 % B in 20 Min.; Flußrate 140 ml/Min.).

Peptid-Fraktionen, die beim Monitorieren mittels analytischer HPLC eine Reinheit größer 99 % und dabei keine Verunreinigung größer 0,5 % zeigen, werden vereinigt. Diese vereinigten Fraktionen werden mit einem Volumenäquivalent deionisiertem Wasser verdünnt und auf das zuvor mit 1000 ml Puffer B3 gereinigte und anschließend mit 300 ml Puffer A3 equilibrierte Biotage-Modul aufgepumpt. Zur Entsalzung wird mit 1000 ml Puffer A3 gespült.

Das Reinprodukt wird durch Spülen der Säule mit 1500 ml Puffer B3 eluiert und das Acetonitril abgedampft. Die verbleibende Lösung wird lyophilisiert.

Es resultieren zwischen 1,7 und 2,2 g hochreines Cardiodilatin-Fragment ANP(99-126). Dieses Fragment wird analog zu dem in Beispiel 14 c) beschriebenen Verfahren in das entsprechende Acetat-Salz umgewandelt. Es resultieren zwischen 1,3 und 1,7 g hochreines ANP(99-126)-Acetat.

### Beispiel 17:

### Reinigung von ANP(102-126) und Herstellung der hochreinen Form

### a) Aufkonzentrierung des cyclisierten Cardiodilatin-Fragmentes ANP(102-126)

Analog zu Beispiel 15 a) wird die Cyclisationslösung (ca. 18 Liter 5 % AcOH, in deionisiertem Wasser (v/v), mit einem Peptidgehalt von ca. 65 g) auf eine mit 1000 ml Puffer A3 (0,1 % TFA (v/v) in deionisiertem Wasser) equilibrierte Glassäule aufgetragen (Flußrate 130 ml/Min.). Nach beendetem Aufpumpen wird das Peptid durch kontinuierliches Zudosieren von Puffer B3 (0,1 % TFA in deionisiertem Wasser/ACN 2:3 v/v) in einem kontinuierlichen Gradienten eluiert (0 % Puffer B während 40 Min.; 15-35 % Puffer B in 90 Min.; 35 % B während 10 Min.; Flussrate 130 ml/Min.). Peptid-Fraktionen, die beim Monitorieren mittels analytischer HPLC eine Reinheit größer 75% zeigen, werden vereinigt. Diese vereinigten Fraktionen werden mit einem Volumenäquivalent deionisiertem Wasser verdünnt und auf ein mit 300 ml Puffer A3 equilibriertes Biotage-Modul aufgetragen (Flußrate 140 ml/Min.). Anschließend wird das konzentrierte Peptid durch Spülen der Säule mit 100% Puffer B3 eluiert und das Acetonitril abgedampft. Die verbleibende Lösung wird lyophilisiert.

Es resultieren zwischen 19 und 23 g des Cardiodilatin-Fragmentes ANP(102-126) mit einer Reinheit größer 90%.

### b) Reinigung des aufkonzentrierten ANP(102-126)

4,8 g des nach Beispiel 17 a) aufkonzentrierten Cardiodilatin-Fragmentes werden in 200 ml 10 % AcOH, in deionisiertem Wasser (v/v), gelöst und auf ein mit 300 ml Puffer A4 (50 mM TEAP pH 2,25 in deionisiertem Wasser) equilibriertes Biotage-Modul aufgetragen (Flußrate 140 ml/Min.). Das Peptid wird durch kontinuierliches Zudosieren von Puffer B4 (50 mM TEAP pH 2,25 in deionisiertem Wasser /ACN 2:3 v/v) in einem kontinuierlichen Gradienten eluiert (22-28 % B in 90 Min.; 28 % B während 10 Min.; 28-40 % B in 20 Min.; Flußrate 140 ml/Min.).

Peptid-Fraktionen, die beim Monitorieren mittels analytischer HPLC eine Reinheit größer 99 % und dabei keine Verunreinigung größer 0,5 % zeigen, werden vereinigt. Diese vereinigten Fraktionen werden mit einem Volumenäquivalent deionisiertem Wasser verdünnt und auf das zuvor mit 1000 ml Puffer B3 gereinigte und anschließend mit 300 ml Puffer A3 equilibrierte Biotage-Modul aufgepumpt. Zur Entsalzung wird mit 1000 ml Puffer A3 gespült.

Das Reinprodukt wird durch Spülen der Säule mit 1500 ml Puffer B3 eluiert und das Acetonitril abgedampft. Die verbleibende Lösung wird lyophilisiert.

Es resultieren zwischen 1,9 und 2,4 g hochreines Cardiodilatin-Fragment ANP(102-126). Dieses Fragment wird analog zu dem in Beispiel 14 c) beschriebenen Verfahren in das entsprechende Acetat-Salz umgewandelt. Es resultieren zwischen 1,5 und 1,9 g hochreines ANP(102-126)-Acetat.

### Beispiel 18:

### Analytische HPLC-Untersuchungen am Beispiel von ANP(95-126)

### a) Elution mit TEAP-Puffer, pH 2,25

Auf einer analytischen HPLC-Säule wurden 50 µg ANP(95-126) injiziert. Als Elutionsmittel diente ein linearer Gradient von Puffer B von 25-45 % in 20 Minuten (Puffer A: 50 mM TEAP, pH 2,25; Puffer B: Mischung von A und Acetonitril im Volumenverhältnis 2:3). Aus dem Chromatogramm aus Abb. 5 ist ersichtlich, daß zwei polare Verunreinigungen enthalten sind, die durch das verwendete Elutionsmittel aufgetrennt werden können.

### Legende zu Abb. 5:

25 - 45 % B in 20 Min.
Puffer A: 50 mM TEAP pH 2,25
Puffer B: A:ACN (2:3)
215 nm 1,0 ml/ C-Nr. 4040465 C
M+N 250 /1/4° /3 Nuc 300 A 5 u C18
D-2500
Method: 50 µg; TAG 243 CH : 1; Peak reject: 5000
File: 1; Calculation method: area%; Table: 0; conc: area

| No. | RT | Area | % | BC |
|---|---|---|---|---|
| 5 | 7,82 | 53358 | 0,311 1 | BV |
| 6 | 8,08 | 84196 | 0,491 | W |
| 7 | 9,07 | 386602 | 2,255 | W |
| 8 | 9,78 | 1265799 | 7,384 | W |
| 9 | 10,56 | 4701290 | 27,430 | W |
| 10 | 10,92 | 10557085 | 61,582 | W |
| 11 | 11,91 | 27613 | 0,161 | TBB |
| 12 | 12,82 | 8763 | 0,051 | TBB |
| 13 | 13,76 | 14346 | 0,084 BB | |
| 14 | 14,86 | 31959 | 0,186 | BB |
| 15 | 19,04 | 10892 | 0,064 | BB |
| Total | | 17143003 | 100,00 | |

### b) Elution mit 0,1 % TFA (Trifluoressigsäure)

Analog zu Beispiel 18 a) werden 50 µg ANP(95-126) der gleichen Herstellungscharge auf eine analytische HPLC-Säule aufgetragen. Als Elutionsmittel diente ein linearer Gradient von Puffer B von 30 - 50 % in 20 Minuten (Puffer A: 0,1 % TFA in Wasser; Puffer B: Mischung von A und Acetonitril im Volumenverhältnis 2:3). Aus dem Chromatogramm aus Abb. 6 ist ersichtlich, daß eine mit Hilfe dieses Elutionsmittels durchgeführte Auftrennung der enthaltenen Verunreinigungen nicht erfolgt. Der Hauptpeak ist im Vergleich zu dem Chromatogramm in Beispiel a) breiter und das isolierte Produkt enthält die beiden polaren Verunreinigungen, die im Chromatogramm nach Abb. 5 erkennbar sind.

### Legende zu Abb. 6:

30 - 50 % B in 20 Min.
Puffer A: 0,1 % TFA in Wasser
Puffer B: A:ACN (2:3)
215 nm 1,0 ml/'C-Nr. 4011079 C
M+N 250 / l/ 4 ° /3 Nuc 300 A 5u C 18
D-2500
Method: 50 µg; TAG 142; CH : 1; Peak reject: 5000
File: 2; Calculation method: area%; Table: 0; conc: area

| No. | RT | Area | % | BC |
|---|---|---|---|---|
| 2 | 3,64 | 5073 | 0,040 | BB |
| 4 | 5,10 | 6624 | 0,053 | BB |
| 5 | 5,92 | 8161 | 0,065 | BB |
| 6 | 7,36 | 6814 | 0,054 | BB |
| 7 | 9,11 | 252878 | 2,012 | BB |
| 9 | 11,73 | 87629 | 0,697 | BB |
| 10 | 12,60 | 258273 | 2,055 | BB |
| 11 | 13,09 | 4578590 | 36,428 | W |
| 12 | 13,26 | 7175177 | 57,086 | W |
| 13 | 14,67 | 179155 | 1,425 | TBB |
| 14 | 17,48 | 10611 | 0,084 | BB |
| Total | | 12568985 | 100,00 | |

### Beispiel 19:

### Prüfung der Reinheit durch Kapillarelektrophorese

Lyophilisierte Proben der hergestellten Endprodukte der Cardiodilatin-Fragmente aus den Beispielen 15 bis 17 werden mit Wasser zu einer Konzentration von 1 mg/ml gelöst und sofort analysiert. Die Kapillarelektrophorese wurde mit dem System P/ACE 2100 von Beckmann unter folgenden Bedingungen durchgeführt:
Kapillare: Fused Silica von Supelco, Trennlänge 50 cm, Innendurchmesser 75 µm Detektionswellenlänge: 200 nm
Injektionszeit: 1s
Trennpuffer: 100 mM Natriumphosphat, pH 2,5; 0,02% Hydroxypropylmethylcellulose Trennparameter: 25°C, 80 µA, 30 min.

Abbildung 3 zeigt das erhaltene Chromatogramm für Urodilatin des Standes der Technik, Abbildung 4 zeigt das Chromatogramm für hochreines Urodilatin, das nach Beispiel 15 erhalten wurde.

Aus dem Vergleich der beiden Chromatogramme geht hervor, daß sich das erfindungsgemäße Urodilatin von dem Urodilatin des Standes der Technik deutlich unterscheidet. Das erfindungsgemäße Urodilatin ist frei von Peptidverunreinigungen.

### ABKÜRZUNGSVERZEICHNIS

### Aminosäuren

- Ala: L-Alanin
- Asn: L-Asparagin
- Asp: L-Asparaginsäure
- Arg: L-Arginin
- Cys: L-Cystein
- Gln: L-Glutamin
- Gly: Glycin
- Ile: L-Isoleucin
- Leu: L-Leucin
- Met: L-Methionin
- Phe: L-Phenylalanin
- Pro: L-Prolin
- Ser: L-Serin
- Thr: L-Threonin
- Tyr: L-Tyrosin

### Schutzgruppen

- Boc: tert. Butyloxycarbonyl
- Fmoc: 9-Fluorenylmethyloxycarbonyl
- OtBu: tert. Butylester
- Pbf: 2,2,4,6,7-pentamethyldihydrobenzofuran-5-sulfonyl
- Pmc: 2,2,5,7,8-Pentamethylchroman-6-sulphonyl
- tBu: tert. Butylether
- Acm: Acetamidomethyl
- Trt: Trityl

### Reagenzien/Lösungsmittel

- ACN: Acetonitril
- TFA: Trifluoressigsäure
- TEAP: Triethylammoniumphosphat

## Patentansprüche

1. Verfahren zur Herstellung von Cardiodilatin-Fragmenten der Formel I
R¹-ANP(105-121)-R² (I),
mit einer Kettenlänge von insgesamt 17 - 37 Aminosäuren, in der ANP(105-121) die Aminosäuresequenz [SEQ ID NO. 1] darstellt,
R¹ eine Aminosäurekette der Sequenz ANP(90-104) [SEQ ID NO, 2] oder einem Fragment davon mit einer Kettenlänge von 0 - 15 Aminosäuren darstellt, und
R² eine Aminosäurenkette der Sequenz ANP(122-126) [SEQ ID NO. 3] oder einem Fragment davon mit einer Kettenlänge von 0 - 5 Aminosäuren darstellt,
**dadurch gekennzeichnet, daß** die Synthese über eine Kondensation von mindestens drei Teilfragmenten erfolgt, wobei die Kondensation der Teilfragmente zum Cardiodilatin-Fragment der Formel [zwischen der Aminosäureposition Gly¹⁰⁸ und Arg¹⁰⁹ und der Aminosäureposition Gly¹²⁰ und Cys¹²¹ durchgeführt wird und über das Teilfragment ANP(109-120) erfolgt.

2. Verfahren nach Anspruch 1, wobei
(a) in einem ersten Schritt die Kondensation der Teilfragmente zwischen den Aminosäurepositionen Gly¹²⁰ und Cys¹²¹ aus den Teilfragmenten ANP(109120) und Cys¹²¹ - R² durchgeführt wird, und
(b) in einem zweiten Schritt die Kondensation der Teilfragmente zwischen den Aminosäurepositionen Gly¹⁰⁸ und Arg¹⁰⁹ aus dem nach Schritt (a) erhaltenen Teiffragment ANP(109-121)-R² und dem Teilfragment R¹ -ANP( 105-108) durchgeführt wird.

3. Verfahren nach einem der Ansprüche 1 oder 2, **dadurch gekennzeichnet, daß** R¹ die Aminosäuresequenz ausgewählt aus der Gruppe ANP(95-104), ANP(99-104) und ANP(102-104) darstellt, und R² die Aminosäuresequenz ausgewählt aus der Gruppe ANP(122-125) und ANP (122-126) darstellt.

4. Verfahren zur Herstellung von Cardiodilatin-Fragmenten R¹-ANP(105-121)-R2 mit einer Kettenlänge von insgesamt 17 - 37 Aminosäuren, nach einem der Ansprüche 1-3 wobei R¹ eine Aminosäurekette der Sequenz ANP(90-104) oder Fragmente davon mit einer Kettenlänge von 0 - 15 Aminosäuren darstellt, und R² eine Aminosäurenkette der Sequenz ANP(122-126) oder Fragmente davon mit einer Kettenlänge von 0 - 5 Aminosäuren darstellt, **dadurch gekennzeichnet, daß** die Reinigung des Rohproduktes mittels einer Umkehrphasen-HPLC-Säule erfolgt und das Cardiodilatin-Fragment mit einem Puffer-system enhaltend Triethylammonium-Phosphat und Acetonitril eluiert wird.

5. Geschützte Fragmente des ANP (95-126) ausgewählt aus der Gruppe mlt folgender Struktur:

6. Verwendung von Peptidfragmenten ausgewählt aus der Gruppe der Aminosäuresequenzen
a) R¹-ANP(105-108), wobei R¹ eine Aminosäurekette der Sequenz ANP(90-104) oder ein Fragment davon mit einer Kettenlänge von 0 - 15 Aminosäuren darstellt,
b) ANP(109-120),
c) ANP(109-121)-R², wobei R² eine Aminosäurenkette der Sequenz ANP(122-126) oder ein Fragment davon mit einer Kettenlänge von 0 - 5 Aminosäuren darstellt, und
d) Cys¹²¹-R², wobei R² eine Aminosäurenkette der Sequenz ANP(122-126) oder ein Fragment davon mit einer Kettenlänge von 3 - 5 Aminosäuren darstellt,
sowie deren durch Schutzgruppen modifizierten Derivate in einem Verfahren zur Herstellung von Cardiodilatin-Fragmenten nach einem der Ansprüche 1 - 3.

## Claims

1. A process for preparing cardiodilatin fragments of formula I
R¹-ANP(105-121)-R² (I)
having a total chain length of from 17 to 37 amino acids, wherein ANP(105-121) represents the amino acid sequence [SEQ ID NO. 1];
R¹ represents an amino acid chain of the sequence ANP(90-104) [SEQ ID NO. 2] or a fragment thereof having a chain length of from 0 to 15 amino acids; and
R² represents an amino acid chain of the sequence ANP(122-126) [SEQ ID NO. 3] or a fragment thereof having a chain length of from 0 to 5 amino acids;
**characterized in that** the synthesis is effected through a condensation of at least three partial fragments, wherein the condensation of the partial fragments to the cardiodilatin fragment of formula I is performed between amino acid positions Gly¹⁰⁸ and Arg¹⁰⁹ and between amino acid positions Gly¹²⁰ and Cys¹²¹ and is effected through the partial fragment ANP(109-120).

2. The process according to claim 1, wherein
(a) in a first step, the condensation of the partial fragments between amino acid positions Gly¹²⁰ and Cys¹²¹ is performed from the partial fragments ANP(109-120) and Cys¹²¹-R²; and
(b) in a second step, the condensation of the partial fragments between amino acid positions Gly¹⁰⁸ and Arg¹⁰⁹ is performed from the partial fragments ANP(109-121)-R² obtained from step (a) and the partial fragment R¹-ANP(105-108).

3. The process according to any of claims 1 or 2, **characterized in that** R¹ represents an amino acid sequence selected from the group consisting of ANP(95-104), ANP(99-104) and ANP(102-104), and R² represents an amino acid sequence selected from the group consisting of ANP(122-125) and ANP(122-126).

4. The process for preparing cardiodilatin fragments R¹-ANP(105-121)-R² having a total chain length of from 17 to 37 amino acids according to any of claims 1 to 3, wherein R¹ represents an amino acid chain of the sequence ANP(90-104) or fragments thereof having a chain length of from 0 to 15 amino acids, and R² represents an amino acid chain of the sequence ANP(122-126) or fragments thereof having a chain length of from 0 to 5 amino acids, **characterized in that** the purification of the raw product is effected by means of a reversed-phase HPLC column, and the cardiodilatin fragment is eluted with a buffer system containing triethylammonium phosphate and acetonitrile.

5. Protected fragments of ANP(95-126) selected from the group of fragments having the following structures:

6. Use of peptide fragments selected from the group of amino acid sequences:
a) R¹-ANP(105-108), wherein R¹ represents an amino acid chain of the sequence ANP(90-104) or a fragment thereof having a chain length of from 0 to 15 amino acids;
b) ANP(109-120);
c) ANP(109-121)-R², wherein R² represents an amino acid chain of the sequence ANP(122-126) or a fragment thereof having a chain length of from 0 to 5 amino acids; and
d) Cys¹²¹-R², wherein R² represents an amino acid chain of the sequence ANP(122-126) or a fragment thereof having a chain length of from 3 to 5 amino acids;
and their derivatives modified by protective groups in a process for preparing cardiodilatin fragments according to any of claims 1 to 3.

## Revendications

1. Procédé de préparation de fragments de cardiodilatine de formule I
R¹-ANP(105-121)-R² (I),
comportant au total un enchaînement de 17 à 37 acides aminés, dans laquelle ANP(105-121) est la séquence d'acides aminés [SEQ. ID. N° 1],
R¹ est une chaîne d'acides aminés de séquence ANP(90-104) [SEQ ID N° 2] ou un fragment de celle-ci ayant une longueur de chaîne de 0 à 15 acides aminés, et
R² est une chaîne d'acides aminés de séquence ANP(122-126) [SEQ ID N° 3] ou un fragment de celle-ci ayant une longueur de chaîne de 0 à 5 acides aminés,
**caractérisé en ce que** la synthèse s'effectue par une condensation d'au moins trois fragments partiels, dans laquelle la condensation de ces fragments partiels en fragments de cardiodilatine de formule I s'effectue entre la position d'acide aminé Gly¹⁰⁸ et Arg¹⁰⁹ et la position d'acide aminé Gly¹²⁰ et Cys¹²¹, à partir du fragment partiel ANP(109-120).

2. Procédé selon la revendication 1, dans lequel
(a) au cours d'une première étape, on effectue la condensation des fragments partiels entre les positions d'acides aminés Gly¹²⁰ et Cys¹²¹ à partir des fragments partiels ANP(109-120) et Cys¹²¹ - R², et
(b) au cours d'une seconde étape, on effectue la condensation des fragments partiels entre les positions d'acides aminés Gly¹⁰⁸ et Arg¹⁰⁹ à partir du fragment partiel ANP(109-121)-R² obtenu après l'étape (a) et du fragment partiel R¹-ANP(105-108).

3. Procédé selon une des revendications 1 ou 2, **caractérisé en ce que** R¹ est une des séquences d'acides aminés sélectionnées parmi ANP(95-104), ANP(99-104) et ANP(102-104), et R² est une des séquences d'acides aminés sélectionnées parmi ANP(122-125) et ANP(122-126).

4. Procédé de préparation de fragments de cardiodilatine R¹-ANP(105-121)-R² comportant au total un enchaînement de 17 à 37 acides aminés selon une des revendications de 1 à 3, dans lequel R¹ est une chaîne d'acides aminés de séquence ANP(90-104) ou des fragments de celle-ci comportant un enchaînement de 0 à 15 acides aminés, et R² est une chaîne d'acides aminés de séquence ANP(122-126) ou des fragments de celle-ci comportant un enchaînement de 0 à 5 acides aminés,
**caractérisé en ce que** la purification du produit brut s'effectue par HPLC sur colonne à phase inversée et **en ce que** le fragment de cardiodilatine est élué par un système tampon contenant du triéthylammonium phosphate et de l'acétonitrile.

5. Fragments protégés de l'ANP (95-126) sélectionnés parmi la série de composés ayant la structure suivante:

6. Utilisation de fragments de peptide sélectionnés dans le groupe formé par les séquences d'acides aminés suivantes:
a) R¹-ANP(105-108), dans laquelle R¹ est une chaîne d'acides aminés de séquence ANP(90-104) ou un fragment de celle-ci comportant un enchaînement de 0 à 15 acides aminés,
b) ANP(109-120),
c) ANP(109-121)- R² dans laquelle R² est une chaîne d'acides aminés de séquence ANP(122-126) ou un fragment de celle-ci comportant un enchaînement de 0 à 5 acides aminés
d) Cys¹²¹- R², dans laquelle R² est une chaîne d'acides aminés de séquence ANP(122-126) ou un fragment de celle-ci comportant de 3 à 5 acides aminés, ainsi que de leurs dérivés modifiés par des groupes protecteurs par un procédé de préparation de fragments de cardiodilatine selon une des revendications de 1 à 3.
